Europäisches Patentamt

European Patent Office

Office européen des brevets

(19)

(11) Veröffentlichungsnummer: **0 247 998 B1**

## (12) EUROPÄISCHE PATENTSCHRIFT

(45) Veröffentlichungstag der Patentschrift: **04.08.93**

(51) Int. Cl.⁵: **A61K 39/395**

(21) Anmeldenummer: **87890116.4**

(22) Anmeldetag: **21.05.87**

(54) Verfahren zur Inaktivierung von vermehrungsfähigen filtrierbaren Krankheitserregern.

(30) Priorität: **30.05.86 AT 1457/86**

(43) Veröffentlichungstag der Anmeldung:
**02.12.87 Patentblatt 87/49**

(45) Bekanntmachung des Hinweises auf die
Patenterteilung:
**04.08.93 Patentblatt 93/31**

(84) Benannte Vertragsstaaten:
**AT BE CH DE ES FR GB IT LI LU NL SE**

(56) Entgegenhaltungen:
**EP-A- 0 120 835**
**FR-A- 2 506 616**

**CHEMICAL ABSTRACTS, Band 98, Nr. 18, 2. Mai 1983, Seite 369, Zusammenfassung Nr. 149490r, Columbus, Ohio, US; E. TABOR et al.: "Inactivation of hepatitis B virus by three methods: treatment with pepsin, urea, or formalin", & J. MED. VIROL. 1983, 11(1), 1-9**

**CHEMICAL ABSTRACTS, Band 83, Nr. 9., 1. September 1975, Seite 153, Zusammenfassung Nr. 72630s, Columbus, Ohio, US; J.T. ENRIGHT et al.: "Disinfection of liquid and aerosol viral systems using immobilized enzymes", & ENVIRON. SCI. TECHNOL. 1975,**

9(6), 586-8

(73) Patentinhaber: **IMMUNO Aktiengesellschaft**
**Industriestrasse 67**
**A-1221 Wien(AT)**

(72) Erfinder: **Eibl, Johann, Dr.**
**Gustav Tschermakgasse 2**
**A-1180 Wien(AT)**
Erfinder: **Linnau, Yendra, Dr.**
**Lavendelweg 24**
**A-1220 Wien(AT)**

(74) Vertreter: **Wolfram, Gustav, Dipl.-Ing.**
**Patentanwälte Sonn, Pawloy, Weinzinger & Wolfram, Riemergasse 14**
**A-1010 Wien (AT)**

EP 0 247 998 B1

## Beschreibung

Die Erfindung betrifft ein Verfahren zur Inaktivierung von vermehrungsfähigen filtrierbaren Krankheitserregern in therapeutisch oder prophylaktisch anzuwendenden Immunglobulin-G-haltigen Blutfraktionen, gegebenenfalls unter Anwendung von erhöhter Temperatur.

Es ist eine umfangreiche Literatur vorhanden, die sich mit der Inaktivierung von vermehrungsfähigen filtrierbaren Krankheitserregern in Blutprodukten befaßt. Die verschiedenen Verfahren umfassen u.a.:

- Erhitzen der Blutprodukte in wässeriger Lösung, gegebenenfalls unter Zusatz stabilisierender Substanzen,
- Behandeln der Blutprodukte mit organischen Lösungsmitteln,
- Erhitzen der Blutprodukte in trockenem Zustand.

Das Bestreben bei diesen Inaktivierungsverfahren geht dahin, die potentielle Infektiosität der Präparationen aufzuheben, ihre biologische Aktivität aber weitgehend zu erhalten. Dieses Ziel wurde bisher nicht in zufriedenstellender Weise erreicht. Vor allem sind diese Verfahren bei Immunglobulinhältigen Lösungen nicht bzw. schlecht anwendbar.

Im einzelnen sind zum Stand der Technik beispielsweise die folgenden Literaturstellen anzuführen:

Die europäische Patentanmeldung 0 139 975 betrifft ein Verfahren zur Pasteurisierung von Humanplasma, wobei eine Plasmalösung in Gegenwart von Calciumionen und von Saccharose auf eine Temperatur bis zu 70°C erhitzt wird.

Die EP-B1 - 0 053 338 beschreibt ein Verfahren zur Inaktivierung von Hepatitis-Viren in Faktor IX und X enthaltenden Präparationen, wobei eine Erwärmung der wässerigen Lösung eines Blutpräparates in Gegenwart von Calciumionen und gegebenenfalls einer Aminosäure und/oder eines Saccharides oder Zuckeralkoholes bei Temperaturen von bis zu 100°C vorgenommen wird.

In der EP-A2 - 0 035 204 wird ein Verfahren zur Inaktivierung von wässerigen Proteinlösungen, die Faktor VIII, Fibronectin, Globulin, Fibrinogen und andere Proteine enthalten können, beschrieben, wobei die Komposition mit einem Polyol gemischt und die Mischung auf eine Temperatur von 60 bis 75°C erhitzt wird.

In der EP-A2 - 0 077 870 ist ein Inaktivierungsverfahren beschrieben, bei welchem eine wässerige, Faktor VIII enthaltende Lösung mit Aminosäuren, Monosacchariden, Oligosacchariden, Zuckeralkoholen und Kohlenwasserstoff- oder Hydroxylkohlenwasserstoffcarbonsäuren mit 3 bis 10 Kohlenstoffatomen auf eine Temperatur von 50 bis 80°C erhitzt wird.

In der PCT-Anmeldung WO 83/04371 ist ein Verfahren zum Inaktivieren von Hepatitis-Viren beschrieben, wobei eine das Virus enthaltende Präparation bei einer Temperatur von 4 bis 40°C mit einem Halogenkohlenwasserstoff, insbesondere Chloroform, behandelt wird.

Die veröffentlichte PCT-Anmeldung WO 82/03871 beschreibt ein Verfahren zur Behandlung von Blutgerinnungsenzyme enthaltenden Zubereitungen, wobei diese zur Inaktivierung vorhandener infektiöser Viren im trockenen Zustand, gegebenenfalls unter Zusatz von Stabilisatoren, wie z.B. Aminosäuren und/oder Zucker, erhitzt werden; als trockener Zustand wird ein solcher mit weniger als 5 Gew.% (0,05) Wasser definiert.

Im japanischen Dokument 51-118825 ist ein Verfahren zur thermischen Stabilisation von IgA (Immunglobulin A) und IgM (Immunglobulin M) beschrieben, wobei durch Hitzebehandlung bei 60°C der Immunglobulin-haltigen Lösung in Gegenwart von neutralen Aminosäuren und Monosacchariden Hepatitis-Viren inaktiviert werden soll.

Schließlich ist noch auf die europäische Patentanmeldung 0 122 909 der Anmelderin zu verweisen, worin ein Verfahren zur Herstellung einer intravenös verabreichbaren Immunglobulin-G-hältigen Fraktion beschrieben ist. Bei diesem Verfahren wird eine Immunglobulin-G-hältige Fraktion mit an wasserunlösliches Trägermaterial gebundenen Pankreasenzymen behandelt, mit dem Ziel, Verunreinigungen, die eine vasoaktive bzw. leukopenische Wirkung und damit Unverträglichkeitsreaktionen zur Folge haben, zu entfernen. Über Inaktivierungswirkungen gegenüber Krankheitserregern wird in dem Dokument nichts berichtet.

Obwohl, wie aus dem referierten Stand der Technik ersichtlich ist, eine große Anzahl von Inaktivierungsverfahren vorgeschlagen worden ist, haben alle diese Verfahren gewisse Nachteile, wie nicht zufriedenstellende Ausbeute und/oder Reduktion der biologischen Wirksamkeit oder keine vollständige Inaktivierung aller in Frage kommenden Viren; bzw. sind sie für Immunglobulin-G-haltige Präparationen in flüssiger Phase nicht anwendbar, ohne einen der genannten Nachteile aufzuweisen.

Die Erfindung bezweckt die Vermeidung dieser Nachteile bzw. Schwierigkeiten und stellt sich die Aufgabe, ein Inaktivierungsverfahren für Immunglobulin-G-hältige Präparationen zur Verfügung zu stellen, bei welchem die Sicherheit gegeben ist, daß bei deren Applikation keine pathogenen Viren mehr enthalten sind, und die Aktivität der Blutprodukte ausreichend voll erhalten bleibt.

Die Erfindung, mit der diese Aufgabe gelöst wird, besteht in der Verwendung von neutralen Peptidhydrolasen zur Inaktivierung von vermehrungsfähigen filtrierbaren Krankheitserregern in therapeutisch oder prophylaktisch anzuwendenden Immunglobulin-G-hältigen Fraktionen, wobei eine wässrige Lösung einer aus menschlichem Blut gewonnenen Immunglobulin-G-haltigen Fraktion bei einer Temperatur von 4 bis 50°C und bei einem pH-Wert von 5,5 bis 9,5 mit den neutralen Peptidhydrolasen behandelt wird.

Als neutrale Peptidhydrolase können ein oder mehrere Enzyme aus der Gruppe der Peptidhydrolasen, wie Trypsin, Chymotrypsin, Carboxypeptidasen, verwendet werden.

Bei Anwendung der Erfindung zur Herstellung von Immunglobulin-G-Präparationen wird bevorzugt nach der Inaktivierung das bzw. die Enzyme aus der Lösung abgetrennt und das behandelte Produkt einer weiteren Reinigung und Konzentrierung unterworfen.

Nach einer Ausführungsform der Erfindung wird die wässerige Lösung der Immunglobulin-G-hältigen Fraktion mit einer löslichen neutralen Peptidhydrolase bei einem PH-Wert von 6,0 bis 8,0, vorzugsweise 7,0 ± 0,4, behandelt.

Gemäß einer anderen Ausführungsform der Erfindung wird die wässrige Lösung der Immunglobulin-G-haltigen Fraktion mit einer an wasserunlöslichem Trägermaterial gebundenen (immobilisierten) neutralen Peptidhydrolase bei einem pH-Wert von 5,5 bis 8,5 behandelt.

Gemäß einer vorteilhaften Ausführungsform wird die Behandlung der Immunglobulin-G-haltigen Fraktion bei erhöhter Temperatur während einer Dauer von 1 Stunde bis 36 Tage durchgeführt.

Die Proteinkonzentration der Immunglobulin-G-haltigen Fraktion kann von 0,1 bis 18 Gew.% betragen.

Von den vermehrungsfähigen filtrierbaren Krankheitserregern, die mit dem erfindungsgemäßen Verfahren zuverlässig inaktiviert werden können, sind besonders Hepatitisviren oder HTLV-III/LAV-Viren (Human T Lymphotropic Virus) hervorzuheben.

Da die notwendigen Untersuchungen über Hepatitis-Virusinaktivierung in Blutprodukten nicht sofort beim Menschen vorgenommen werden können und Schimpansen nur in ungenügender Zahl zur Verfügung stehen, erfolgt erfindungsgemäß die Bewertung der Wirksamkeit der Inaktivierung mit Hilfe von Modellviren.

Das erfindungsgemäße Inaktivierungsverfahren sowie die Herstellung einer Immunglobulin-G-haltigen Präparation unter Anwendung des Verfahrens, die erreichten Wirkungen und die Überlegenheit gegenüber bekannten Verfahren sind in den folgenden Beispielen und Tabellen näher erläutert.

Beispiel 1:

a) Herstellung einer Immunglobulin-G-hältigen Fraktion:

Menschliches Blutplasma wird bei einem pH-Wert von 7,2 und einer Temperatur von -2°C mit 8 % Äthanol versetzt. Nach Abtrennen des Präzipitates wird die Äthanolkonzentration auf 25 % erhöht und gleichzeitig die Temperatur auf -6°C gesenkt. Der Niederschlag, der Immunglobulin-G enthält, wird durch Extraktion mit einem Phosphat-Acetat-Puffer weiter gereinigt und anschließend bei einem pH-Wert von 5,4 und einer Temperatur von -2°C mit 12 % Äthanol versetzt. Der Niederschlag wird verworfen. Die Äthanolkonzentration des Überstandes wird, bei einem pH-Wert von 7,2 und einer Temperatur von -8°C, auf 25 % erhöht. Das ausgefallene pastenförmige Immunglobulin wird gesammelt und das Äthanol durch Dialyse, Gefriertrocknung oder Ultrafiltration entfernt.

Die Immunglobulin-G-haltige Fraktion wird auf einen Proteingehalt von 10 % eingestellt und durch Filtration sterilisiert.

b) Inaktivierung von Vacciniavirus mit immobilisiertem Trypsin:

Das in diesem Beispiel verwendete immobilisierte Trypsin wurde in folgender Weise bereitet:

1 l Sepharose 4 B-Gel (Pharmacia) wurde nach einer Waschung mit 4 l destilliertem Wasser mit 200 g Bromcyan, die in 100 ml Acetonitril gelöst wurden, bei einem pH-Wert von 11,0 versetzt. Das Reaktionsgemisch wurde durch ein Eisbad gekühlt. Nach Entfernung der flüssigen Phase wurde das Gel mit 800 mg Trypsin (Sigma), das in 1 l 0,2 molarem $NaHCO_3$ gelöst wurde, versetzt. Das nicht gebundene Trypsin wurde vom Trypsin, das an das Gel gebunden ist, durch Filtrieren getrennt.

Nachdem das immobilisierte Trypsin mit 1 l einer 1 molaren Glycinlösung versetzt wurde, wurde es gründlich mit 0,2 molarer $NaHCO_3$-Lösung proteinfrei gewaschen. Schließlich wurde es in 1 l 0,9 %iger NaCl-Lösung suspendiert - es ist gebrauchsfertig für die Inkubation mit einer Immunoglobulinfraktion.

10 ml der nach lit.a) erhaltenen Immunglobulinlösung wurden mit 1 ml des wie oben beschriebenen immobilisierten Trypsins und mit 0,5 ml einer Vaccinia Virus Suspension (Vaccinia Virus, ATCC VR-862, Virusstamm Elstree, American Type Culture Collection) versetzt und unter sterilen Bedingungen bei 37°C unter Rühren behandelt.

Eine Vergleichslösung, enthaltend 10 ml der Immunglobulinlösung gemäß a), 1 ml immobilisiertes Trypsin und 0,5 ml virusfreies Medium, wurde zur Bestimmung der IgG Monomeren und der biologischen Aktivität vorbereitet und in gleicher Weise wie die virushaltige Lösung (gleiche Temperatur, gleiche Rührdauer) behandelt.

Zu Beginn und nach verschiedenen Zeitintervallen, nämlich nach 24 h, 48 h und 75 h, wurden Proben der virushältigen Lösung entnommen und der Virustiter bestimmt. Dies erfolgte in folgender Weise:

Die virushaltige Lösung wurde mit isotoner Kochsalzlösung im Verhältnis 1 : 10 seriell verdünnt. Der Titer des Virus wurde durch Bewertung des zytopathischen Effektes auf sensitive Vero-Zellen in der Mikrotiterplatte bestimmt. Die Ergebnisse wurden nach statistischer Behandlung der Auswertung entsprechend der Formel von Reed und Muench als Logarithmus $TCID_{50}$ ausgedrückt (Reed J.L. and H. Muench; Amer.J.Hyg. 27, 493 - 497, (1938)).

Die erhaltenen Virustiter sind aus Tabelle 1 zu entnehmen, woraus sich ergibt, daß der zu Beginn der Untersuchung vorhandene Virustiter von 2,6 nach 24 h auf 1,0 und nach 48 h auf kleiner als 1,0 gesunken ist.

c) Bestimmung der biologischen Aktivität, u.zw. der Gehalt an Tetanus Antikörper in IE/ml und des Anteiles an IgG Monomeren in virusfreien Proben:

Die Bestimmung der Tetanus Antikörper beruht darauf, daß eine bestimmte Menge an Tetanus Toxin mit verschiedenen Mengen an Tetanusantitoxin-hältigen Proben gemischt und nach vorheriger Inkubation Mäusen injiziert wird. Aufgrund der auftretenden Todesraten werden im Vergleich zum WHO Standard die Internationalen Einheiten/ml ermittelt. (Europ. Pharmakopoeia, 2nd Edition, Part.II-2, p. 91-91-3, 1981).

Die Bestimmung des Gehaltes an IgG Monomeren erfolgte mittels HPLC (High Performance Liquid Chromatography), indem die IgG-haltige Vergleichslösung einer HPLC-Analyse unterworfen wurde. Als Trennsäule fand eine Bio Sil TSK® 250-Säule, 600 × 7,5 mm, für einen Molekulargewichtsbereich von 1.000 bis 300.000 Anwendung. Als Elutionsmittel wurde ein Natriumdehydrogenphosphat-Natriumsulfat-Puffer, pH 6,8, verwendet. Als Monomerengehalt wurde der Peak mit Werten für Ve/Vo 1,28 - 1,67 herangezogen (T. Tomono et al., Analytical Biochemistry, 123: 394 - 401, 1982).

Beispiel 2:

Inaktivierung von Sindbisvirus mit immobilisiertem Trypsin.

10 ml einer in gleicher Weise wie in Beispiel 1 hergestallten Immunglobulin-G-haltigen Lösung wurden mit 1 ml immobilisiertem Trypsin und 0,5 ml einer Sindbisvirus Suspension (ATCC VR-68, Virusstamm AR 339, American Type Culture Collection) versetzt und unter sterilen Bedingungen bei 37°C unter Rühren behandelt. Als Vergleichslösung zur Aktivitätsbestimmung diente die gleiche Lösung wie in Beispiel 1.

Zu Beginn und nach verschiedenen Zeitintervallen wurden Proben entnommen und der Virustiter bestimmt.

Die Ergebnisse sind aus Tabelle 2 zu ersehen: Nach 75 h ist der Sindbisvirustiter, ausgehend von 4,5 um 2,6 log Stufen auf 1,9 abgefallen. Die Gehalte an Tetanus Antikörpern und der IgG-Monomeren entsprechen jenen Werten, die in Tabelle 1 angegeben sind.

Beispiel 3:

Inaktivierung von HTLV-III$_B$ (Human T Lymphotropic Virus III$_B$) mit immobilisiertem Trypsin.

10 ml einer in gleicher Weise wie in Beispiel 1 hergestellten Immunglobulin-G-haltigen Lösung wurde mit 1 ml immobilisiertem Trypsin und 0,5 ml einer HTLV-III$_B$ Suspension (R.C. Gallo et al, Science 224: 500 - 503, 1984) versetzt und unter sterilen Bedingungen bei 37°C unter Rühren behandelt. Zu Beginn und nach verschiedenen Zeitintervallen wurden Proben entnommen und die Virus-Aktivität festgestellt. Die Bestimmung der Virusaktivität "Infektiöse Einheiten/0,5 ml" erfolgte nach der in der obigen Literaturstelle R. C. Gallo et al angegebenen Methode. Als Vergleichslösung zur biologischen Aktivitätsbestimmung diente eine Vergleichslösung wie in Beispiel 1.

Die Virus-Aktivitäten und der Anteil an IgG Monomeren in der Vergleichslösung wird aus Tabelle 3 ersichtlich: nach 48 h ist die Virus-Aktivität verschwunden, der IgG Monomerenanteil zum größten Teil erhalten.

Beispiel 4:

Inaktivierung von Vacciniavirus mit löslicher Hydrolase.

1 ml einer wie in Beispiel 1 hergestellten Immunglobulin-G-hältigen Lösung wurde mit 0,1 ml einer 7,5 %igen Hydrolaselösung (Trypsin-Pankreasprotease, Merck Artikel 8367) und mit 0,1 ml einer Vacciniavirussuspension (Vaccinia Virus, ATCC VR-862, Virusstamm Elstree, American Type Culture Collection) versetzt und unter sterilen Bedingungen bei 37°C behandelt. Eine Vergleichslösung aus 1 ml Immunglobulinlösung, 0,1 ml Hydrolaselösung und 0,1 ml virusfreiem Medium wurde vorbereitet und in gleicher Weise wie die virushaltige Lösung behandelt. Nach Probenahme zu verschiedenen Zeiten wurden der Virustiter, der Gehalt an Tetanusantikörpern und der Anteil der IgG Monomeren, wie beschrieben, bestimmt. Die Ergebnisse sind aus Tabelle 4 zu entnehmen. Nach 48 Stunden betrug der Virustiter weniger als 1 bei ausreichendem IgG-Monomeranteil.

Beispiel 5:

Inaktivierung von Sindbisvirus mit löslicher Hydrolase.

1 ml einer wie in Beispiel 1 hergestellten Immunglobulin-G-haltigen Lösung wurde mit 0,1 ml einer 7,5 %igen Hydrolaselösung (Trypsin-Pankreasprotease) und 0,1 ml einer Sindbisvirussuspension (ATCC VR-68, Virusstamm Elstree, American Type Culture Collection) versetzt und unter sterilen Bedingungen bei 37 °C behandelt. Eine Vergleichslösung aus 1 ml Immunglobulinlösung, 0,1 ml Hydrolaselösung und 0,1 ml virusfreiem Medium wurde vorbereitet und in gleicher Weise wie die virushältige Lösung behandelt. Nach Probenahme zu verschiedenen Zeiten wurden der Virustiter, der Gehalt an Tetanusantikörpern und der Anteil der IgG Monomeren, wie beschrieben, bestimmt. Die Ergebnisse sind aus Tabelle 5 zu entnehmen. Nach 48 h betrug der Virustiter weniger als 1 bei ausreichendem IgG Monomeranteil.

Beispiel 6:

Inaktivierung von HTLV-III$_B$ mit löslicher Hydrolase.

1 ml einer wie in Beispiel 1 hergestellten Immunglobulin-G-haltigen Lösung wurde mit 0,1 ml einer 7,5 %igen Hydrolaselösung (Trypsin-Pankreasprotease) und 0,1 ml einer HTLV-III$_B$ Virussuspension (R.C. Gallo et al) versetzt und unter sterilen Bedingungen bei 37 °C behandelt. Eine Vergleichslösung aus 1 ml Immunglobulinlösung, 0,1 ml Hydrolaselösung und 0,1 ml virusfreiem Medium wurde vorbereitet und in gleicher Weise wie die virushältige Lösung behandelt. Nach Probenahme zu verschiedenen Zeiten wurden die Virus-Aktivität, die Tetanusantikörper und der Anteil der IgG Monomeren, wie beschrieben, bestimmt. Die Ergebnisse sind aus Tabelle 6 zu entnehmen. Nach 72 h war die Virus-Aktivität verschwunden, bei ausreichendem IgG Monomeranteil und zufriedenstellendem Antikörpergehalt.

Während die vorgenannten Beispiele die erreichbaren Inaktivierungsergebnisse unter Zugabe von bestimmten Modellviren erläutern, wird im Produktionsprozeß ohne Viruszugabe gearbeitet. Die Abtrennung des Inaktivierungsenzyms, was eine bevorzugte Ausführungsform darstellt, erfolgt hierbei nach der Rührstufe bei den angegebenen Temperaturen durch ausgewählte Maßnahmen, u.zw. z.B. bei Verwendung eines immobilisierten Enzyms durch Filtration und bei Verwendung eines löslichen Enzyms durch Adsorption an Ionenaustauschern oder Aluminiumhydroxid.

Tabelle 1

Behandlung mit immobilisiertem Trypsin

| nach Beginn der Behandlung | Virus Titer (log TCID$_{50}$/0,1 ml) Vacciniavirus | Tetanus Antikörper (IE/ml) | IgG Monomere (%) |
|---|---|---|---|
| vor Beginn der Behandlung | 2,6 | 90 | 90,9 |
| 24 Stunden | 1,0 | 80 | 82,2 |
| 48 Stunden | kleiner als 1,0 | 60 | 75,7 |
| 75 Stunden | kleiner als 1,0 | 52 | 69,4 |

EP 0 247 998 B1

## Tabelle 2

| Behandlung mit immobilisiertem Trypsin nach | Virus Titer (log $TCID_{50}/0{,}1$ ml) Sindbisvirus | Tetanus Antikörper (IE/ml) | IgG Monomere (%) |
|---|---|---|---|
| vor Beginn der Behandlung | 4,5 | 90 | 90,9 |
| 24 Stunden | 3,1 | 80 | 82,2 |
| 48 Stunden | 2,5 | 60 | 75,7 |
| 75 Stunden | 1,9 | 52 | 69,4 |

## Tabelle 3

| Behandlung mit immobilisiertem Trypsin nach | Virus Aktivität (Infektiöse Einheiten/0,5 ml) $HTLV-III_B$ Virus | IgG Monomere (%) |
|---|---|---|
| vor Beginn der Behandlung | $10^6$ | 89,4 |
| 1 Stunde | $10^4$ | 89,9 |
| 24 Stunden | $10^0$ | 90,8 |
| 48 Stunden | 0 | 76,8 |
| 72 Stunden | 0 | 71,2 |

Tabelle 4

| Behandlung mit löslichen Hydrolasen nach | Virus Titer (log $TCID_{50}/0,1$ ml) Vaccinia Virus | Tetanus Antikörper (IE/ml) | IgG Monomere (%) |
|---|---|---|---|
| vor Beginn der Behandlung | 2,1 | 90 | 82,9 |
| 5 Stunden | 1,2 | 90 | 83,5 |
| 24 Stunden | 1,0 | 70 | 66,1 |
| 48 Stunden | kleiner als 1,0 | 65 | 52,5 |
| 72 Stunden | kleiner als 1,0 | 55 | 41 |

Tabelle 5

| Behandlung mit löslichen Hydrolasen nach | Virus Titer (log $TCID_{50}/0,1$ ml) Sindbisvirus | Tetanus Antikörper (IE/ml) | IgG Monomere (%) |
|---|---|---|---|
| vor Beginn der Behandlung | 5,0 | 90 | 82,9 |
| 5 Stunden | 4,5 | 90 | 83,5 |
| 24 Stunden | 1,2 | 70 | 66,1 |
| 48 Stunden | kleiner als 1,0 | 65 | 52,5 |
| 72 Stunden | kleiner als 1,0 | 55 | 41 |

Tabelle 6

| Behandlung mit löslichen Hydrolasen nach | Virus Aktivität (Inf.Einheit/0,5 ml) HTLV-IIIB | Tetanus Antikörper (IE/ml) | IgG Monomere (%) |
|---|---|---|---|
| vor Beginn der Behandlung | $10^5$ | 90 | 82,9 |
| 5 Stunden | $10^4$ | 90 | 83,5 |
| 24 Stunden | $10^2$ | 70 | 66,1 |
| 48 Stunden | $10^1$ | 65 | 52,5 |
| 72 Stunden | 0 | 55 | 41 |

## Patentansprüche

1. Verwendung von neutralen Peptidhydrolasen zur Inaktivierung von vermehrungsfähigen filtrierbaren Krankheitserregern in therapeutisch oder prophylaktisch anzuwendenden Immunglobulin-G-haltigen Fraktionen, wobei eine wässrige Lösung einer aus menschlichem Blut gewonnenen Immunglobulin-G-haltigen Fraktion bei einer Temperatur von 4 bis 50 °C und bei einem pH-Wert von 5,5 bis 9,5 mit den neutralen Peptidhydrolasen behandelt wird.

2. Verwendung nach Anspruch 1, wobei als neutrale Peptidhydrolase ein oder mehrere Enzyme aus der Gruppe der Peptidhydrolasen, wie Trypsin, Chymotrypsin, Carboxypeptidasen, verwendet werden.

3. Verwendung nach Anspruch 1 oder 2, wobei nach der Inaktivierung das bzw. die Enzyme aus der Lösung abgetrennt und das behandelte Produkt einer weiteren Reinigung und Konzentrierung unterworfen wird.

4. Verwendung nach Anspruch 1 oder 2, wobei die wässrige Lösung der Immunglobulin-G-haltigen Fraktion mit einer löslichen neutralen Peptidhydrolase bei einem pH-Wert von 6,0 bis 8,0 behandelt wird.

5. Verwendung nach Anspruch 1 oder 2, wobei die wässrige Lösung der Immunglobulin-G-haltigen Fraktion mit einer an wasserunlöslichem Trägermaterial gebundenen (immobilisierten) neutralen Peptidhydrolase bei einem pH-Wert von 5,5 bis 8,5 behandelt wird.

6. Verwendung nach einem oder mehreren der Ansprüche 1 bis 5, wobei die Behandlung der Immunglobulin-G-haltigen Fraktion bei erhöhter Temperatur während einer Dauer von 1 Stunde bis 36 Tage durchgeführt wird.

7. Verwendung nach einem oder mehreren der Ansprüche 1 bis 6, wobei die Proteinkonzentration der Immunglobulin-G-haltigen Fraktion von 0,1 bis 18 Gew.% beträgt.

8. Verwendung nach einem oder mehreren der Ansprüche 1 bis 7, wobei die vermehrungsfähigen filtrierbaren Krankheitserreger Hepatitisviren oder HTLV-III/LAV-Viren (Human T Lymphotropic Virus) sind.

## Claims

1. The use of neutral peptide hydrolases for inactivating reproductive filterable pathogens in immunoglobulin-G-containing fractions to be applied therapeutically or prophylactically, wherein an aqueous solution of an immunoglobulin-G-containing fraction obtained from human blood is treated with said neutral peptide hydrolases at a temperature of from 4 to 50 °C and at a pH of from 5.5 to 9.5.

2.  The use according to claim 1, wherein one or several enzymes from the group consisting of peptide hydrolases, such as trypsin, chymotrypsin, carboxypeptidases, are used as said neutral peptide hydrolase.

3.  The use according to claim 1 or 2, wherein said enzyme(s) are separated from the solution upon inactivation and the treated product is subjected to further purification and concentration.

4.  The use according to claim 1 or 2, wherein the aqueous solution of the immunoglobulin-G-containing fraction is treated with a soluble neutral peptide hydrolase at a pH of from 6.0 to 8.0.

5.  The use according to claim 1 or 2, wherein the aqueous solution of the immunoglobulin-G-containing fraction is treated with an (immobilized) neutral peptide hydrolase bound to water-insoluble carrier material at a pH of from 5.5 to 8.5.

6.  The use according to one or several of claims 1 to 5, wherein the treatment of the immunoglobulin-G-containing fraction is carried out at elevated temperature for a period of from 1 hour to 36 days.

7.  The use according to one or several of claims 1 to 6, wherein the protein concentration of the immunoglobulin-G-containing fraction is 0.1 to 18 % by weight.

8.  The use according to one or several of claims 1 to 7, wherein the reproductive filterable pathogens are hepatitis viruses or HTLV-III/LAV viruses (human T lymphotropic virus).

**Revendications**

1.  Utilisation de peptide-hydrolases neutres pour l'inactivation de germes pathogènes filtrables, susceptibles de multiplication, dans des fractions contenant des immunoglobulines à utiliser à des fins thérapeutiques ou prophylactiques, une solution aqueuse d'une fraction contenant des immunoglobulines, obtenue à partir de sang humain, étant traitée par des peptide-hydrolases neutres à une température de 4 à 50 °C et à un pH de 5,5 à 9,5.

2.  Utilisation selon la revendication 1, une ou plusieurs enzymes du groupe des peptide-hydrolases, telles que trypsine, chymotrypsine, carboxypeptidases, étant utilisées comme peptide-hydrolase neutre.

3.  Utilisation selon la revendication 1 ou 2, la ou les enzymes étant séparées de la solution après l'inactivation et le produit traité étant soumis à une purification et concentration supplémentaires.

4.  Utilisation selon la revendication 1 ou 2, la solution aqueuse de la fraction contenant des immunoglobulines étant traitée par une peptide-hydrolase neutre soluble à un pH de 6,0 à 8,0.

5.  Utilisation selon la revendication 1 ou 2, la solution aqueuse de la fraction contenant des immunoglobulines G étant traitée par une peptidehydrolase neutre liée à un véhicule insoluble dans l'eau (immobilisée) à un pH de 5,5 à 8,5.

6.  Utilisation selon une ou plusieurs des revendications 1 à 5, le traitement de la fraction contenant des immunoglobulines G étant opéré à une température élevée pendant une durée de 1 h à 36 jours.

7.  Utilisation selon une ou plusieurs des revendications 1 à 6, la concentration en protéines de la fraction contenant des immunoglobulines G étant comprise entre 0,1 et 18 % en poids.

8.  Utilisation selon une ou plusieurs des revendications 1 à 7, les germes pathogènes filtrables susceptibles de multiplication étant des virus de l'hépatite ou des virus HTLV-III/LAV (Human T Lymphotropic Virus).